# EUROPEAN PATENT APPLICATION

(11) **EP 2 431 465 A1**
(43) Date of publication of application: **21.03.2012**
(21) Application number: 10774902.0
(22) Date of filing: 11.05.2010
(51) Int. Cl.: C12N 15/09

(54) **METHOD FOR SYNTHESIS OF DOUBLE-STRANDED DNA CORRESPONDING TO RNA, AND METHOD FOR AMPLIFICATION OF THE DNA**

(30) Priority: 14.05.2009 JP 2009117151
(71) Applicant: Wako Pure Chemical Industries, Ltd., Osaka-shi Osaka 540-8605 (JP)
(72) Inventor: HAYASHIDA Yukinobu, Amagasaki-shi Hyogo 661-0963 (JP)
(74) Representative: von Kreisler Selting Werner
(86) International application number: PCT/JP2010/057942
(87) International publication number: WO 2010/131645

(57) **Abstract**

An object of the present invention is to provide an inexpensive and simple method for synthesis of a double-stranded DNA corresponding to a particular RNA, and a method for amplification of the aforementioned double-stranded DNA. The present invention relates to a method for synthesis of a double-stranded DNA having a nucleotide sequence corresponding to template RNA having polyA, comprising step 1 in which reverse transcription reaction of template RNA is carried out employing oligo(dT)primer to which DNA fragment having a known sequence has been added at the 5'-terminal, to obtain a single-stranded DNA, and step 2 in which double strand formation reaction of single-stranded DNA obtained in step 1 is carried out employing a random primer to which DNA fragment having a known sequence has been added at the 5'-terminal, in the presence of polymerase which does not have 3'→ 5' exonuclease activity nor strand displacement activity, to obtain a double-stranded DNA, as well as a method for amplification of a double-stranded DNA having a nucleotide sequence corresponding to template RNA having polyA, further comprising step 3 in which PCR reaction is carried out using the obtained double-stranded DNA.

## Description

### TECHNICAL FIELD

The present invention relates to a method for synthesis of a double-stranded DNA corresponding to a particular RNA and a method for amplification of the aforementioned double-stranded DNA.

### BACKGROUND ART

In the conventional gene analysis, for the purpose of obtaining full-length cDNA, various studies have been carried out including SMART method, Oligo-Capping method, Cap Trapper method, and the like. However, all of these methods had met such problems that full length cDNA cannot be obtained except the case where RNA having both polyA and Cap structure is used, and RNA fragment defective in Cap structure cannot be analyzed, and the like. On the other hand, although the microarray method is known as a method which can analyze RNA fragment as well, the aforementioned method had not been easily used in the experimental level, because the method requires expensive equipment.

Therefore, development of a new method, which is capable of performing gene analysis even if RNA fragment is defective in Cap structure, and enables easy and cheap gene analysis, has been desired.

### SUMMARY OF INVENTION

### PROBLEM TO BE SOLVED BY THE INVENTION

An object of the present invention is to provide a method for synthesis of a double-stranded DNA from a particular RNA and a method for amplification of the aforementioned double-stranded DNA.

### MEANS FOR SOLVING THE PROBLEM

In view of the above-described situation, the present inventors have studied intensively on a method for obtaining cDNA fragment of RNA and a gene analysis of RNA using cDNA fragment thereof, focusing on the fact that cDNA can be identified from the database if a part of fragment can be analyzed even not for full-length cDNA, because databases of many cDNAs have now been accumulated. As a result, the inventors have found that a cDNA fragment corresponding to a RNA having polyA can be obtained at low cost and easily, by synthesizing a first strand from RNA having polyA such as mRNA and a fragment derived from mRNA employing oligo(dT)primer to which DNA fragment having a known sequence has been added at 5'-terminal, and then synthesizing a second strand from the first strand, employing an adapter-added random primer, in the presence of polymerase which does not have 3'→5' exonuclease activity nor strand displacement activity, and thus completed the present invention.
The present invention relates to:
"a method for synthesis of a double-stranded DNA having a nucleotide sequence corresponding to template RNA having polyA, comprising: step 1 in which reverse transcription reaction of template RNA is carried out employing oligo(dT)primer to which DNA fragment having a known sequence has been added at the 5'-terminal, to obtain a single-stranded DNA; and step 2 in which double strand formation reaction of the single-stranded DNA obtained in step 1 is carried out employing a random primer to which DNA fragment having a known sequence has been added at the 5'-terminal, in the presence of polymerase which does not have 3'→5' exonuclease activity nor strand displacement activity, to obtain a double-stranded DNA", and "a method for amplification of a double-stranded DNA having a nucleotide sequence corresponding to template RNA having polyA, comprising step 1 in which reverse transcription reaction of template RNA having polyA is carried out employing an oligo(dT)primer to which DNA fragment having a known sequence (adapter 1) has been added at the 5'-terminal, to obtain a single-stranded DNA; step 2 in which double strand formation reaction of the single-stranded DNA obtained in step 1 is carried out employing a random primer to which DNA fragment having a known sequence (adapter 2) has been added at the 5'-terminal, in the presence of polymerase which does not have 3'→5' exonuclease activity nor strand displacement activity, to obtain a double-stranded DNA; and step 3 in which PCR reaction is carried out by using the double-stranded DNA obtained in step 2 as a template".

### EFFECT OF THE INVENTION

According to the present invention, even though a sequence of template RNA having polyA is unknown, a double-stranded DNA having a corresponding (complementary) nucleotide sequence can be obtained. In other words, it becomes possible to obtain a double-stranded DNA corresponding to a RNA fragment which does not have Cap structure, though it had been difficult by a conventional method. In addition, it becomes also possible to obtain a double-stranded DNA corresponding to a RNA having polyA without requiring any expensive equipment such as microarray method. Therefore, according to the method of the present invention, easy and cheap gene analysis of RNA becomes possible. Specifically, simple and cheap identification of a template RNA becomes possible, by analyzing a sequence of the double-stranded DNA obtained by the method of the present invention and comparing it with the known database.
Furthermore, according to the method for amplification of the present invention, it becomes possible to obtain a sufficient amount of DNA for cloning even though RNA having polyA is in a trace amount. Moreover, since chain length of a double-stranded DNA to be obtained does not depend on chain length of the RNA having polyA, it becomes possible to amplify while an existence ratio of RNA having polyA to total RNA in the sample is maintained, and also to apply to quantitative analysis of RNA becomes possible.

### BRIEF DESCRIPTION OF DRAWINGS

[Fig. 1]

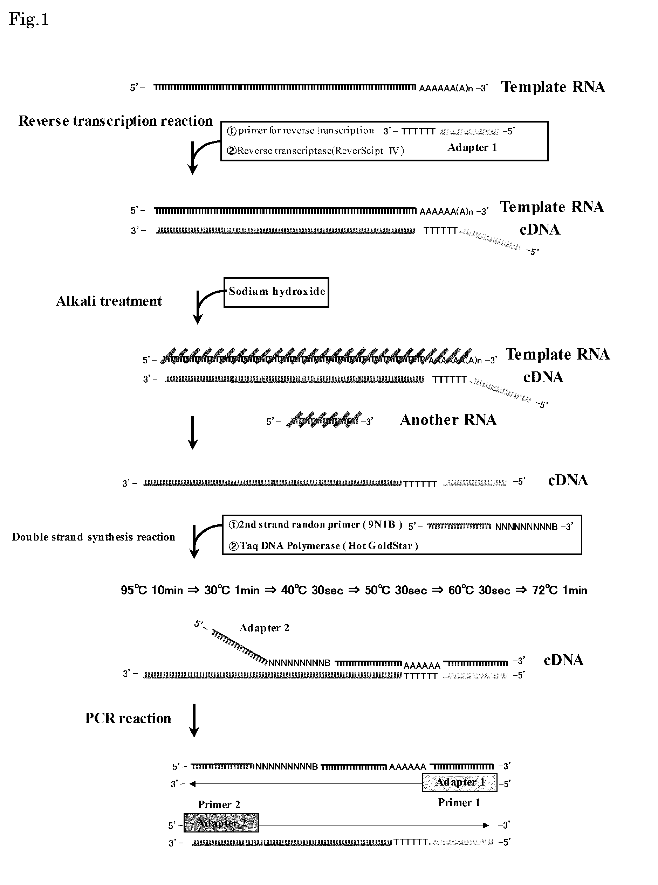

   Fig. 1 shows a pattern diagram illustrating an example of the method for amplification of a double-stranded DNA of the present invention.
[Fig. 2]

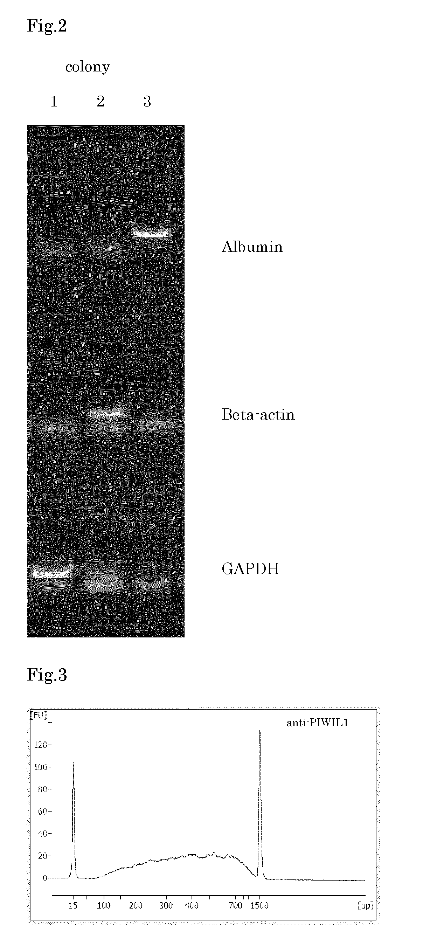

   Fig. 2 shows an image of the gel after electrophoresis of the inserted fragments of 3 kinds of the same clones obtained by colony PCR in Example 1, 6.
[Fig. 3]
   Fig. 3 is a result of capillary electrophoresis carried out using Agilent 2100 Bioanalyzer, using a DNA which was synthesized according to the present invention from RNA as a template, which was obtained by immunoprecipitation employing a mouse anti-PIWIL1 antibody-immobilized carrier and mouse testis as a sample, in Example 4.
[Fig. 4]

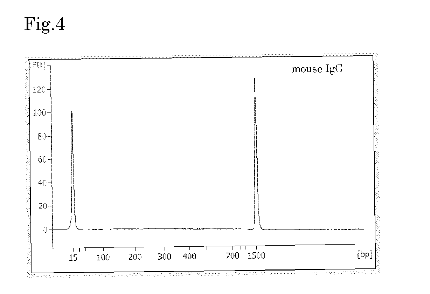

   Fig. 4 is a result of capillary electrophoresis carried out using Agilent 2100 Bioanalyzer, using a DNA which was synthesized according to the present invention from RNA as a template, which was obtained by immunoprecipitation employing a mouse anti-IgG antibody-immobilized carrier and mouse testis as a sample, in Example 4.

### EMBODIMENTS FOR CARRYING OUT THE INVENTION

The template RNA having polyA relevant to the present invention (hereinafter, sometimes simply referred to as template RNA relevant to the present invention) is not particularly limited, so long as it is a RNA having polyA, and specifically includes, for example, messenger RNA (mRNA), non-coding RNA, Alu RNA, and the like. In addition, the template RNA relevant to the present invention may be a fragment of these RNAs, and for example, may be a defective one in Cap structure. It should be noted that although synthesis of a double-stranded DNA having a nucleotide sequence corresponding to the RNA defective in this Cap structure was difficult by the conventional method other than microarray, even in the case of such RNA, a corresponding double-stranded DNA can be synthesized easily by the present invention. In addition, the template RNA relevant to the present invention may be either the one of known sequence or the one of unknown sequence, and one of the effects of the present invention is that even in the case of the one of unknown sequence, a corresponding double-stranded DNA can be synthesized and amplified. Chain length of the template RNA relevant to the present invention is usually 30 bases or more to 1500 bases, preferably 30 to 1000 bases, more preferably 30 to 100 bases, and further more preferably 30 to 50 bases.

The double-stranded DNA obtained by the method for synthesis of double-stranded DNA of the present invention represents a single-stranded DNA containing a part of the nucleotide sequence corresponding to the template RNA relevant to the present invention and a single-stranded DNA corresponding to that single-stranded DNA.

### Method for Synthesis of a double-stranded DNA having a nucleotide sequence corresponding to RNA having poly A

The method for synthesis of a double-stranded DNA having a nucleotide sequence corresponding to RNA having polyA (hereinafter, sometimes simply referred to as method for synthesis of double-stranded DNA of the present invention) is comprising:
1) step 1 in which reverse transcription reaction of template RNA is carried out employing oligo(dT)primer to which DNA fragment having a known sequence (hereinafter, sometimes simply referred to as adapter 1) has been added at the 5'-terminal, to obtain a single-stranded DNA; and
2) step 2 in which double strand formation reaction of single-stranded DNA obtained in Step 1 is carried out employing random primer to which DNA fragment having a known sequence has been added at the 5'-terminal, in the presence of polymerase which does not have 3'→5' exonuclease activity nor strand displacement activity, to obtain a double-stranded DNA.

The above-described step 1 is carried out according to the conventional method usually used in this field, employing oligo(dT)primer to which adapter 1 is added at the template RNA having polyA relevant to the present invention, and thereby a single-stranded DNA containing a nucleotide sequence complementary to the template RNA relevant to the present invention is obtained. This step can be also carried out using a commercially available kit, but it may be carried out, for example, according to the method described in Nucleic Acids Research, 1988, Vol. 16, No. 5 1999-2014 and Nucleic Acids Research, 1988, Vol. 16, No. 1 265-277. Specifically, for example, the step 1 is carried out by adding oligo(dT)primer to which adapter 1 has been added, reverse transcriptase, a mixture of 4 deoxyribonucleotide triphosphates (dNTPs) to template RNA, and reacting in the buffer solution such as Tris buffer solution (pH 8.3) usually at 35 to 50°C, and preferably at 40 to 50°C for usually 5 to 40 minutes, and preferably for 5 to 20 minutes, and then terminating the reaction by heat-treatment or addition of reaction-terminating liquid. Thereby, a single-stranded DNA complementary to the template RNA relevant to the present invention can be obtained.

Amount of the template RNA relevant to the present invention to be used in this case varies depending on a sample to be used, and therefore, is not particularly limited, but an amount of nucleic acid of the template RNA relevant to the present invention is usually 1 ng to 1 µg. For example, in the case in which a total RNA is used, an amount of the sample is usually 100 ng to 50 µg, and preferably 1 to 20 µg. It should be noted that these RNAs are usually subjected to the above-described step with the above amount of nucleic acid being contained in the solution, and amount of the solution is usually 1 to 30 µL, and preferably 1 to 20 µL. As a solvent of the sample solution containing such RNA, sterile distilled water is usually used.

The above-described oligo(dT)primer is not particularly limited, so long as the primer binds to polyA, and all of those usually used in this field can be employed. Specifically, the oligo(dT)primer is usually 10 to 100mer, preferably 10 to 50mer, and more preferably 15 to 30mer. Also, the DNA fragment having a known sequence to be added to oligo(dT)primer is not particularly limited, so long as the fragment can be used as an adapter, and is usually 10 to 50mer, preferably 12 to 40mer, and more preferably 15 5 to 30mer. Amount of oligo(dT)primer to which adapter 1 has been added to be used is usually 1 to 250 pmol, and preferably 10 to 50 pmol per 1 µg of the nucleic acid amount of the template RNA.

The reverse transcriptase in the above-described reverse transcription reaction is not particularly limited, so long as it is usually used in this field. The reverse transcriptase includes, for example, Moloney Murine Leukemia Virus (M-MLV) reverse transcriptase, Avian myeloblastosis virus (AMV) reverse transcriptase, M-MLV reverse transcriptase (RNase H minus), and the like, and among them, M-MLV reverse transcriptase (RNase H minus) is preferable. In addition, amount thereof to be used varies depending on a type of the enzyme, but is usually 1 to 400 units, and preferably 10 to 200 units per 1 µg of nucleic acid amount of the template RNA.

The above-described dNTPs are a mixture of 4 deoxyribonucleotide triphosphates usually used in this field, and amount thereof to be used is usually 0.1 to 20 nmol, and preferably 1 to 10 nmol per 1 µg of nucleic acid amount of the template RNA. The above-described reaction termination liquid includes, for example, those containing a chelating agent such as EGTA and EDTA. Amount thereof to be used varies depending on a type of the chelating agent, but the liquid is added so that final concentration becomes usually 10 to 100 mmol/L, and preferably 40 to 60 mmol/L. The heat treatment for terminating the reaction is carried out usually at 65 to 100°C, and preferably at 65 to 70°C, and usually for 15 to 60 minutes, and preferably for 15 to 30 minutes. In the above-described reverse transcription reaction, a reagent which is usually employed in such reverse transcription reaction, for example, a reducing agent such as DTT (dithiothreitol), potassium chloride, manganese chloride and ribonuclease inhibitor, may be added, and concentration and amount of these reagents to be used are selected appropriately from the ranges usually employed in this field.

In the above-described step 1, it is preferable to remove the template RNA relevant to the present invention and purify the obtained single-stranded DNA after the step. Specifically, for example, the purification is carried out by subjecting the solution after the reverse transcription reaction in step 1 to alkaline treatment, or to RNaseH treatment. The aforementioned alkaline treatment is carried out, for example, by alkalinizing the reaction solution by adding alkali or an aqueous solution thereof to the solution after the reverse transcription reaction. The aforementioned alkali includes, for example, alkali metal hydroxide such as sodium hydroxide and potassium hydroxide; alkaline earth metal hydroxide such as barium hydroxide, magnesium hydroxide and calcium hydroxide; alkali metal carbonate such as sodium carbonate; ammonia; and amines. Among them, alkali metal hydroxide such as sodium hydroxide and potassium hydroxide is preferable, and above all, sodium hydroxide is particularly preferable. The aforementioned alkaline treatment is specifically carried out by adjusting pH of the aforementioned solution to 10 to 14, and preferably 12 to 14, and heating usually at 50 to 80°C, and preferably at 60 to 70°C, usually for 20 to 120 minutes, and preferably for 20 to 60 minutes. The aforementioned RNaseH treatment is carried out by separating the template RNA relevant to the present invention and the corresponding reversely transcribed DNA by adding RNaseH to the solution after the reverse transcription reaction, thereafter by performing column purification usually carried out in this field. RNaseH employed in this case may be anyone so long as it is those usually used in this field, and may be either the one prepared by the known method or a commercially available one. Further, amount of RNaseH to be used is not particularly limited so long as it is within a range usually employed in this field.

Step 2 is carried out by performing a double strand formation reaction of the single-stranded DNA obtained in step 1, employing a random primer to which DNA fragment having a known sequence (hereinafter, sometimes simply referred to as adapter 2) has been added at 5'-terminal thereof, in the presence of polymerase which does not has 3'→5' exonuclease activity nor strand displacement activity, and thereby, double-stranded DNA of the known sequence corresponding to the template RNA relevant to the present invention can be obtained. The aforementioned step 2 is specifically carried, for example, by adding a random primer to which DNA fragment having a known sequence (adapter 2) has been added at 5'-terminal, polymerase which does not have 3'→5' exonuclease activity nor strand displacement activity, and a mixture of 4 kinds of deoxyribonucleotide triphosphates (dNTPs) to the solution containing the single-stranded DNA obtained in step 1, and reacting usually at 90 to 98°C for 1 to 15 minutes, at 20 to 40°C for 10 seconds to 5 minutes, and at 65 to 75°C for 10 seconds to 10 minutes, in this order.

Chain length of the random primer employed in step 2 is usually 5 to 15mer. Since the random primer of 6mer or shorter is liable to be annealed together with PCR primer when subjected to PCR, and 13me or longer is highly liable to be annealed by the random primers themselves. Therefore, 7 to 12mer is preferable, and 9 to 12me is more preferable. Specifically, when C (cytosine), G (guanine) or T (thymine) is expressed by B, and A (adenine), C (cytosine), G (guanine) or T (thymine) is expressed by N, the random primer includes, for example, 12N, 11N, 10N, 9N, 8N, 7N, 14N1B, 13N1B, 11N1B, 10N1B, 9N1B, 8N1B and the like, and preferably 12N, 11N, 10N, 9N, 11N1B, 10N1B, 9N1B, 8N1B and the like. Among them, 11N1B, 10N1B, 9N1B, 12N, 11N are particularly preferable. DNA fragment having a known sequence (adapter 2) to be added to the random primer is not particularly limited so long as it can be used as the adapter, and chain length thereof is usually 10 to 50mer, preferably 12 to 40mer, and more preferably 15 to 30mer. Amount to be used of the random primer to which adapter 2 has been added is usually 1 to 250 pmol, and preferably 10 to 50pmol per 1 µg of the nucleic acid amount.

Polymerase which does not have 3'→5' exonuclease activity nor strand displacement activity to be employed in step 2 may be the one which does not have both of 3'→5' exonuclease activity and strand displacement activity, and specifically includes, for example, Taq DNA polymerase, Tth DNA polymerase, and the like. Among them, Taq DNA polymerase is preferable. In step 2, when polymerase having 3'→5' exonuclease activity and strand displacement activity is employed, the random primer is annealed to DNA due to the strand displacement activity, and the elongated nucleotide chain is liberated. As a result, by annealing of the random primer to the liberated nucleotide chain, various DNAs are synthesized. However, when polymerase which does not have 3'→5' exonuclease activity nor strand displacement activity is employed, since the random primer which is annealed to DNA and elongated is not liberated, the double-stranded DNA relevant to the present invention can be efficiently synthesized. Amount to be used of polymerase which does not have 3'→5' exonuclease activity nor strand displacement activity varies depending on a type of the enzyme, but is usually 1 to 10 units, and preferably 1 to 5 units per 100 pg to 100 ng of nucleic acid amount.

Four kinds of mixed deoxyribonucleotide triphosphates (dNTPs) to be employed in step 2 may be the one employed in this field as the one to be employed in step 1, and amount to be used is usually 0.1 to 20 nmol, and preferably 1 to 10 nmol per 100 pg to 100ng of the nucleic acid amount of the template DNA.

Double strand formation reaction in step 2 is carried out by reacting usually at 90 to 98°C for 1 to 15 minutes, at 20 to 40°C for 10 seconds to 5 minutes, and at 65 to 75°C for 10 seconds to 10 minutes, and preferably at 94 to 96°C for 1 to 10 minutes, at 25 to 35°C for 1 to 3 minutes, and at 68 to 72°C for 30 seconds to 2 minutes, in this order, and temperature may be varied in a stepwise fashion, so long as the above-described conditions are included. That is, the reaction may be carried out by reacting at 90 to 98°C for 1 to 15 minutes, at 25 to 35°C for 10 seconds to 5 minutes, at 35 to 45°C for 10 seconds to 5 minutes, at 45 to 55°C for 10 seconds to 5 minutes, at 55 to 65°C for 10 seconds to 5 minutes, and at 65 to 75°C for 10 seconds to 10 minutes, in this order.

Preferable examples of the above-described method for synthesis of double-stranded DNA of the present invention are explained as follows.

That is, first of all, a single-stranded DNA complementary to the template RNA can be obtained by adding 1 to 5 µL of a solution containing 10 to 50 µmol/L of oligo(dT)primer to which the known sequence has been added at the 5'-terminal and 1 to 5 µL of a solution containing 10 to 200 units/µL of reverse transcriptase, for example, to 10µL of a solution containing a template RNA having polyA of 1 to 20 ng in converted nucleic acid amount, and reacting them at 40 to 50°C for 10 to 60 minutes. Further, remaining RNA is degraded by adding alkali metal hydroxide such as sodium hydroxide to the solution containing the single-stranded DNA complementary to the template RNA so that pH of the reaction solution becomes 12 to 14, and reacting at 60 to 70°C for 30 to 60 minutes. Subsequently, a single-stranded DNA containing the nucleotide sequence complementary to the template RNA relevant to the present invention can be obtained, by purifying the obtained single-stranded DNA by a purification method using a method such as extraction by column fractionation, and the like.

Further, a double-stranded DNA relevant to the present invention can be obtained by adding 1 to 5 µL of the random primer to which 5 to 20 µmol/L of DNA fragment having a known sequence has been added at the 5'-terminal, as well as 1 to 5 µL of a mixed solution of 4 kinds of deoxyribonucleotide triphosphates (dNTPs) of each 1 to 5 mmol/L, and 1 to 5 units of polymerase which does not have 3'→5' exonuclease activity nor strand displacement activity, for example, to 10 µL of the resulting solution containing the single-stranded DNA relevant to the present invention, and reacting them, for example, (1) at 93 to 98°C for 1 to 10 minutes → (2) at 25 to 35°C for 30 seconds to 5 minutes, (3) at 68 to 72°C for 1 to 5 minute. The resulting double-stranded DNA relevant to the present invention may be purified, for example, by extracting by the column fractionation.

### Method for amplification of double-stranded DNA having a nucleotide sequence corresponding to RNA having poly A

The method for amplification of double-stranded DNA having a nucleotide sequence corresponding to RNA having polyA relevant to the present invention (hereinafter, sometimes simply referred to as method for amplification of double-stranded DNA of the present invention) is comprising the following steps:
1) step 1 in which reverse transcription reaction of template RNA having polyA is carried out employing oligo(dT)primer to which DNA fragment having a known sequence (adapter 1) has been added at the 5'-terminal, to obtain a single-stranded DNA;
2) step 2 in which double strand formation reaction of the single-stranded DNA obtained in step 1 is carried out, employing a random primer to which DNA fragment having a known sequence (adapter 2) has been added at the 5'-terminal in the presence of polymerase which does not have 3'→5' exonuclease activity nor strand displacement activity, to obtain a double-stranded DNA; and
3) step 3 in which PCR reaction is carried out employing the double-stranded DNA obtained in step 2 as a template, employing primer 1 having a nucleotide sequence of adapter 1 at the 3'-terminal and primer 2 having a nucleotide sequence of adapter 2 at the 3'-terminal.

The above-described step 1 and step 2 are the same steps as step 1 and step 2 described in the section of the above-described method for synthesis of a double-stranded DNA of the present invention, and preferable procedures thereof are also same. That is, by subjecting to step 3 the double-stranded DNA relevant to the present invention obtained through the step of the above-described step 1 and step 2, the double-stranded DNA containing a nucleotide sequence corresponding to the template RNA relevant to the present invention can be amplified.

The above-described step 3 is carried out, for example, as follows.

That is, step 3 is carried out, first of all, by preparing primer 1 which has been designed so that adapter 1 (DNA fragment having a known sequence to be added to oligo(dT)primer relevant to the present invention) is contained at the 3'-terminal and primer 2 which has been designed so that adapter 2 (DNA fragment having a known sequence to be added to random primer relevant to the present invention) is contained at the 3'-terminal, and subjecting the double-stranded DNA obtained in step 2 to PCR reaction, by employing these primers 1 and 2 as well as polymerase which does not have 3'→5' exonuclease activity nor strand displacement activity.

The above-described primer 1 contains all of or a part of the sequence of adapter 1, and chain length thereof is usually 12 to 30mer, preferably 15 to 25mer, and more preferably 18 to 22mer. The above-described primer 2 contains all of or a part of the sequence of adapter 2, and chain length thereof is usually 12 to 30mer, preferably 15 to 25mer, and more preferably 18 to 22mer. Amounts of primers 1 and 2 to be used are usually 1 to 250 pmol, and preferably 10 to 50 pmol per 10 pg to 100 ng of the double-stranded DNA obtained in step 2.

The polymerase which does not have 3'→5' exonuclease activity nor strand displacement activity to be employed in step 3 includes the same one as employed in step 2, and preferable amount thereof to be used is the same. It should be noted that in the method for amplification of the present invention, a double-stranded DNA having different chain lengths becomes difficult to be prepared by employing the polymerase which does not have 3'→5' exonuclease activity nor strand displacement activity in step 2 and step 3. Therefore, amplification of a double-stranded DNA becomes possible while an existence ratio of the template RNA relevant to the present invention to the total RNA is maintained.

The above-described PCR reaction may be carried out according to the method well known per se, for example, the method described in Nucleic Acids Research, 1991,Vol. 19,3749, BioTechniques, 1994, Vo1.16, 1134-1137. Specifically, the reaction is carried out as follows. That is, the double-stranded DNA relevant to the present invention can be amplified, for example, by adding the above-described primer 1 (an amount resulting usually 0.1 to 100 pmol, and preferably 0.1 to 50 pmol), the above-described primer 2 (an amount resulting usually 0.1 to 100 pmol, and preferably 0.1 to 50 pmol), a mixed solution of 4 kinds of deoxyribonucleotide triphosphates (dNTPs) (amount each resulting usually 0.01 to 50 nmol, and preferably 0.1 to 20 nmol), and a polymerase which does not have 3'→5' exonuclease activity nor strand displacement activity (an amount of 1 to 5 units) to an aqueous solution containing the double-stranded DNA relevant to the present invention obtained through steps 1 and 2 by employing 1 ng to 100 ng of RNA (20 to 40 µL), and reacting them in a buffer solution such as Tris hydrochloride buffer solution, for example, in a cycle of (1) at 93 to 98°C for 1 to 10 minutes → 2) at 93 to 98°C for 10 to 30 seconds → at 50 to 60°C for 10 to 30 seconds → at 68 to 72°C for 10 to 30 minutes (10 to 30 cycles), (3) at 68 to 72°C for 1 to 5 minutes. In the above-described PCR reaction, after the reaction, the amplified double-stranded DNA is preferably purified by a purification method usually used in this field, for example, a method such as extraction with a mixed solution of phenol / chloroform / isoamyl alcohol, alcohol precipitation, column purification and filter filtration.

It should be noted that a pattern diagram showing an example of the method for amplification of the double-stranded DNA of the present invention is shown in the Fig. 1.

As for the double-stranded DNA relevant to the present invention obtained by the method for synthesis and the method for amplification of double-stranded DNA according to the present invention as described above, nucleotide sequence analysis can be performed by the method usually used in this field as described in Proceedings of the National Academy of Sciences, 1995, Vol 92, 4347-4351 and the like. Specifically, for example, the nucleotide sequence analysis is carried out by transforming the obtained double-stranded DNA to competent cells and the like using a kit for cloning then incubating to amplify by colony PCR, thereafter carrying out plasmid extraction and decoding the nucleotide sequence by a kit using the resulting plasmid as a template. Identification of RNA can be performed by carrying out a homology search using the decoded nucleotide sequence.

Hereinafter, the present invention will be explained in more detail by referring to Examples, Reference Examples, and the like. However, the present invention is not limited by these Examples or the like by no means.

### EXAMPLES

### Example 1: Cloning of 3 kinds of mRNAs (Human Albumin, Beta actin, GAPDH)

### 1. Reverse transcription reaction

An aqueous solution (11 µL) containing 1×10⁹ copies of mRNAs of Human Albumin (2122 bases), Beta actin (1874 bases) and GAPDH (1380 bases) (produced by Nippon Gene Co., Ltd.), respectively, was used as a template RNA aqueous solution. To the aforementioned aqueous solution, a 20 µmol/L primer solution for reverse transcription
(5'-GACCATATGACGAGATCCGAGCTTCTTTTTTTTTTTTTTTTTTTT-3') (1 µL) was added. Subsequently, after heated at 70°C for 3 minutes to cause heat denaturation, the solution was allowed to stand on ice for 2 minutes. Thereafter, a buffer solution for reverse transcription (500 mmol/L Tris-HCl buffer (pH 8.3) containing 750 mmol/L potassium chloride, 30 mmol/L magnesium chloride and 50 mmol/L dithiothreitol) (2 µL), dNTPs (a mixed solution containing 2.5 mmol/L each of dATP, dGTP, dCTP, dTTP solutions, produced by Nippon Gene Co., Ltd.) (4 µL), ribonuclease inhibitor solution (20 units/µL, Ribonuclease Inhibitor (Super), produced by Wako Pure Chemical Industries, Ltd.) (1 µL), and reverse transcriptase solution (200 units/µL, ReverScript IV, produced by Wako Pure Chemical Industries, Ltd.) (1 µL) were added, and the mixture was mixed gently under the ice cooling. After the mixture was reacted at 42°C for 10 minutes, the reaction was terminated by adding 0.5 mol/L EDTA (2 µL) thereto and mixing well.

### 2. Alkali treatment

To the solution after termination of the reaction, 0.2 mol/L NaOH (8 µL) was added and mixed. After the solution was heated at 65°C for 30 minutes, allowed to stand on ice for 2 minutes. Further, after 1 mol/L Tris-HCl (pH 7.5) (20 µL) was added thereto and mixed, unreacted reverse transcription reaction primer was removed using Invisorb Spin PCRapid Kit (produced by Invitek Inc.). Subsequently, Ethachinmate (produced by Nippon Gene Co., Ltd.) (1 µL), 10 mol/L ammonium acetate (12 µL), and ethanol (125 µL) were added, respectively, and mixed. Further, the mixture was centrifuged (18,800 × g) for 10 minutes and washed twice with 70% ethanol, precipitated and dried, and then the reverse transcription reaction product was dissolved in sterilized water (35.5 µL).

### 3. Second strand synthesis reaction

To the aqueous solution containing reverse transcription reaction product (35.5 µL), 10 × Reaction Buffer for HotGoldstar DNA polymerase (produced by EUROGENTEC Inc.) (5 µL), 2.5 mmol/L each of dNTP Mixture (produced by Nippon Gene Co., Ltd.) (4 µL), 20 µmol/L random primer solution for the second strand synthesis (5'-CATGGTATCGACGAGACTGAGGCTGNNNNNNNNNB-3', B represents C, G or T, and N represents A, C, G or T) (1 µL), 25 mmol/L MgCl₂ (4 µL), and HotGoldstar DNA polymerase (5 U/µL, produced by EUROGENTEC Inc.) (0.5µL) were added, respectively, mixed gently on ice, and second strand synthesis reaction was carried out under the following conditions:
95°C for 10 minutes → 30°C for 1 minute → 40°C for 30 seconds → 50°C for 30 seconds → 60°C for 30 seconds → 72°C for 2 minutes → 95°C for 1 minute → on ice for 2 minutes.

Subsequently, unreacted reverse transcription reaction primer was removed from the resulting solution by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125µL) were added, respectively, and mixed. Further, the mixture was centrifuged for 10 minutes (18,800 × g), washed with 75% ethanol twice, and the precipitate was dried and then dissolved in sterilized water (34.5 µL).

### 4.PCR

To the aqueous solution (34.5 µL) obtained in the above item 3, 10 × Reaction Buffer for HotGoldstar DNA polymerase (produced by EUROGENTEC Inc.) (5 µL), 2.5 mmol/L dNTP Mixture (produced by Nippon Gene Co., Ltd.) (4 µL), PCR primer solution (20 µmol/L, 5'-CATGGTATCGACGAGACTGAG-3' or 5'-GACCATATGACGAGATCCGAG-3' is contained) (1 µL each), 25 mmol/L MgCl₂ (4 µL), and HotGoldstar DNA polymerase (5 U/µL, produced by EUROGENTEC Inc.) (0.5 µL) were added, respectively, mixed gently on ice, and PCR reaction was carried out under the following conditions.
95°C for 10 minutes → 95°C for 20 seconds · 60°C for 10 seconds · 72°C for 30 seconds (24 cycles) → 72°C for 2 minutes.

Subsequently, unreacted reverse transcription reaction primer was removed from the resulting solution by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125 µL) were added, respectively, and mixed. Further, the mixture was centrifuged for 10 minutes (18,800 × g), washed with 75% ethanol twice, and the precipitate was dried and then dissolved in sterilized water (4 µL).

### 5. Transformation

To the cDNA-containing solution for cloning (2 µL) obtained in the above item 4, 1 µL of 20 ng/µL of pGEM Teasy Vector (produced by Promega Inc.) and DNA Ligation Kit Mighty Mix solution (produced by Takara Biotech Co., Ltd.) (3 µL) were added, and the mixture was reacted at 16°C for 30 minutes, to insert the cDNA for cloning into the vector. Thereafter, the total volume was transformed into competent cells using ECOS™ Competent E. coli DH5α (produced by Nippon Gene Co., Ltd.) by the heat shock method at 42°C for 45 seconds. Subsequently, the competent cells were incubated in the Luria-Bertani's broth (LB) agar media containing 100 µg/mL of ampicillin sodium at 37°C for 16 hours.

### 6. Colony PCR

A part of each single 48 colonies on the media was added to the reaction solution for Albumin cDNA amplification [total volume (10 µL): 10 × Universal Buffer (produced by Nippon Gene Co., Ltd.) (1 µL), 2.5 mM dNTPs (a mixed solution of 2.5 mmol/L each of dATP, dGTP, dCTP, dTTP, produced by Nippon Gene Co., Ltd.) (1 µL), 5 µmol/L of 2 kinds of primer solutions (5'-GTATCGACGAGACTGAGGCTG-3', 5'-GAAGCACAGAGAAAAGAGGCAAAATG-3') (1 µL each), sterilized water (5.9 µL), Gene Taq NT (5 units/µL, produced by Nippon Gene Co., Ltd.) (0.1 µL)], the mixture was mixed gently on ice, and PCR reaction was carried out under the following conditions. 95°C for 2 minutes → 95°C for 20 seconds · 58°C for 10 seconds · 72°C for 20 seconds (30 cycles) → 72°C for 1 minute.

In addition, for the same 48 colonies, PCR reactions of Beta actin and GAPDH were carried out in the same manner. The reaction solution of Beta actin (total volume:10 µL) was same as the case of Albumin except that the primers for Beta actin amplification (5'-GTATCGACGAGACTGAGGCTG-3', 5'-GTGTGCACTTTTATTCAACTGGTC-3') were used as the primer, and reaction solution of GAPDH (total volume: 10 µL) was same as the case of Albumin except that the primers for GAPDH amplification (5'-GTATCGACGAGACTGAGGCTG-3', 5'-GAGCACAGGGTACTTTATTGATGG-3') were used as the primer.

After that, 6 × Loading Buffer Triple Dye (produced by Nippon Gene Co. Ltd.) (2 µL each) were added to each of the resulting PCR reaction solutions and mixed.

### 7. Electrophoresis

The solution (5 µL) obtained in the above item 6 were subjected to electrophoresis on 1.5% agarose gel, and immersed in 0.5µg/mol ethidium bromide stain solution for 10 minutes, and then a type of insertion fragment in each colony was identified using FAS-III (ultraviolet light irradiator: manufactured by Toyobo Co., Ltd.). Results of the electrophoresis for 3 kinds of clone PCR amplification products in the 1^{st} to 3^{rd} columns are shown in Fig. 2. As a result, it was identified that colony 1 was insertion fragment derived from GAPDH, colony 2 was insertion fragment derived from Beta actin, and colony 3 was insertion fragment derived from Albumin.

In addition, for 48 colonies, it was examined to which of 3 kinds of mRNA each colony corresponds. Results are shown in Table 1.

**[Table 1]**

| mRNA | Number of colony |
|---|---|
| Albumin | 15 |
| Beta actin | 19 |
| GAPDH | 14 |
| | Total 48 |

As a result, it was found that Beta actin : Albumin : GAPDH = 19 : 15: 14. Therefore, the ratio of 3 kinds of mRNA was approximately 1:1:1, and it was found that amplification had been carried out reflecting the existence ratio of mRNA. That is, it was shown that the double-stranded DNA obtained according to the method of the present invention could be amplified while the existence ratio thereof was maintained.

It should be noted that the nucleotide sequence of mRNA used in Example 1 is shown below.

Albumin mRNA, Human (produced by Nippon Gene Co., Ltd.)

GGGUUUAGCUUUUCUCUUCUGUCAACCCCACACGCCUUUGGCACAAUGAA
GUGGGUAACCUUUAUUUCCCUUCUUUUUCUCUUUAGCUCGGCUUAUUCCA
GGGGUGUGUUUCGUCGAGAUGCACACAAGAGUGAGGUUGCUCAUCGGUUU
AAAGAUUUGGGAGAAGAAAAUUUCAAAGCCUUGGUGUUGAUUGCCUUUGC
UCAGUAUCUUCAGCAGUGUCCAUUUGAAGGUCAUGUAAAAUUAGUGAAUG
AAGUAACUGAAUUUGCAAAAACAUGUGUUGCUGAUGAGUCAGCUGAAAAU
UGUGACAAAUCACUUCAUACCCUUUUUGGAGACAAAUUAUGCACAGUUGC
AACUCUUCGUGAAACCUAUGGUGAAAUGGCUGACUGCUGUGCAAAACAAG
AACCUGAGAGAAAUGAAUGCUUCUUGCAACACAAAGAUGACAACCCAAAC

CUCCCCCGAUUGGUGAGACCAGAGGUUGAUGUGAUGUGCACUGCUUUUCA
UGACAAUGAAGAGACAUUUUGAAAAAUACUUAUAUGAAAUUGCCAGA
AGACAUCCUUACUUUUAUGCCCCGGAACUCCUUUCUUUGCUAAAAGGUA
UAAAGCUGCUUUUACAGAAUGUUGCCAAGCUGCUGAUAAAGCUGCCUGCC
UGUUGCCAAAGCUCGAUGAACUUCGGGAUGAAGGGAAGGCUUCGUCUGCC
AAACAGAGACUCAAGUGUGCCAGUCUCCAAAAAUUUGGAGAAAGAGCUUU
CAAAGCAUGGGCAGUAGCUCGCCUGAGCCAGAGAUUUCCCAAAGCUGAGU
UUGCAGAAGUUUCCAAGUUAGUGACAGAUCUUACCAAAGUCCACACGGAA
UGCUGCCAUGGAGAUCUGCUUGAAUGUGCUGAUGACAGGGCGGACCUUGC
CAAGUAUAUCUGUGAAAAUCAAGAUUCGAUCUCCAGUAAACUGAAGGAAU
GCUGUGAAAAACCUCUGUUGGAAAAAUCCCACUGCAUUGCCGAAGUGGAA
AAUGAUGAGAUGCCUGCUGACUUGCCUUCAUUAGCUGCUGAUUUUGUUGA
AAGUAAGGAUGUUUGCAAAAACUAUGCUGAGGCAAAGGAUGUCUUCCUGG
GCAUGUUUUUGUAUGAAUAUGCAAGAAGGCAUCCUGAUUACUCUGUCGUG
CUGCUGCUGAGACUUGCCAAGACAUAUGAAACCACUCUAGAGAAGUGCUG
UGCCGCUGCAGAUCCUCAUGAAUGCUAUGCCAAAGUGUUCGAUGAAUUUA
AACCUCUUGUGGAAGAGCCUCAGAAUUUAAUCAAACAAAAUUGUGAGCUU
UUUGAGCAGCUUGGAGAGUACAAAUUCCAGAAUGCGCUAUUAGUUCGUUA
CACCAAGAAAGUACCCCAAGUGUCAACUCCAACUCUUGUAGAGGUCUCAA
GAAACCUAGGAAAAGUGGGCAGCAAAUGUUGUAAACAUCCUGAAGCAAAA
AGAAUGCCCUGUGCAGAAGACUAUCUAUCCGUGGUCCUGAACCAGUUAUG
UGUGUUGCAUGAGAAAACGCCAGUAAGUGACAGAGUCACCAAAUGCUGCA
CAGAAUCCUUGGUGAACGGGCGACCAUGCUUUUCAGCUCUGGAAGUCGAU
GAAACAUACGUUCCCAAAGAGUUUAAUGCUGAAACAUUCACCUUCCAUGC
AGAUAUAUGCACACUUUCUGAGAAGGAGAGACAAAUCAAGAAACAAACUG
CACUUGUUGAGCUUGUGAAACACAAGCCCAAGGCAACAAAAGAGCAACUG
AAAGCUGUUAUGGAUGAUUUCGCAGCUUUUGUAGAGAAGUGCUGCAAGGC

UGACGAUAAGGAGACCUGCUUUGCCGAGGAGGGUAAAAACUUGUUGCUG
CAAGUCAAGCUGCCUUAGGCUUAUAACAUCACAUUUAAAAGCAUCUCAGC
CUACCAUGAGAAUAAGAGAAAGAAAAUGAAGAUCAAAAGCUUAUUCAUCU
GUUUUUCUUUUUCGUUGGUGUAAAGCCAACACCCUGUCUAAAAAACAUAA
AUUUCUUUAAUCAUUUUGCCUCUUUUCUCUGUGCUUCAAUUAAUAAAAAA
UGGAAAGAAUCUAAAAAAAAAAAAAAAAAAAAAAAAAAAAAAAAAAAAAA
AAAAAAAAAAAAAUAACCCCUUGG.

Beta actin mRNA, Human (produced by Nippon Gene Co., Ltd.)

GGGUUUACCGCCGAGACCGCGUCCGCCCCGCGAGCACAGAGCCUCGCCUUU
GCCGAUCCGCCGCCCGUCCACACCCGCCGCCAGCUCACCAUGGAUGAUGAU
AUCGCCGCGCUCGUCGUCGACAACGGCUCCGGCAUGUGCAAGGCCGGCUUC
GCGGGCGACGAUGCCCCCCGGGCCGUCUUCCCCUCCAUCGUGGGGCGCCCC
AGGCACCAGGGCGUGAUGGUGGGCAUGGGUCAGAAGGAUUCCUAUGUGGG
CGACGAGGCCCAGAGCAAGAGAGGCAUCCUCACCCUGAAGUACCCCAUCG
AGCACGGCAUCGUCACCAACUGGGACGACAUGGAGAAAAUCUGGCACCAC
ACCUUCUACAAUGAGCUGCGUGUGGCUCCCGAGGAGCACCCCGUGCUGCU
GACCGAGGCCCCCCUGAACCCCAAGGCCAACCGCGAGAAGAUGACCCAGAU
CAUGUUUGAGACCUUCAACACCCCAGCCAUGUACGUUGCUAUCCAGGCUG
UGCUAUCCCUGUACGCCUCUGGCCGUACCACUGGCAUCGUGAUGGACUCC
GGUGACGGGGUCACCCACACUGUGCCCAUCUACGAGGGGUAUGCCCUCCCC
CAUGCCAUCCUGCGUCUGGACCUGGCUGGCCGGGACCUGACUGACUACCUC
AUGAAGAUCCUCACCGAGCGCGGCUACAGCUUCACCACCACGGCCGAGCGG
GAAAUCGUGCGUGACAUUAAGGAGAAGCUGUGCUACGUCGCCCUGGACUU
CGAGCAAGAGAUGGCCACGGCUGCUUCCAGCUCCUCCCUGGAGAAGAGCU
ACGAGCUGCCUGACGGCCAGGUCAUCACCAUUGGCAAUGAGCGGUUCCGC
UGCCCUGAGGCACUCUUCCAGCCUUCCUUCCUGGGCAUGGAGUCCUGUGG

CAUCCACGAAACUACCUUCAACUCCAUCAUGAAGUGUGACGUGGACAUCC
GCAAAGACCUGUACGCCAACACAGUGCUGUCUGGCGGCACCACCAUGUAC
CCUGGCAUUGCCGACAGGAUGCAGAAGGAGAUCACUGCCCUGGCACCCAG
CACAAUGAAGAUCAAGAUCAUUGCUCCUCCUGAGCGCAAGUACUCCGUGU
GGAUCGGCGGCUCCAUCCUGGCCUCGCUGUCCACCUUCCAGCAGAUGUGG
AUCAGCAAGCAGGAGUAUGACGAGUCCGGCCCCUCCAUCGUCCACCGCAA
AUGCUUCUAGGCGGACUAUGACUUAGUUGCGUUACACCCUUUCUUGACAA
AACCUAACUUGCGCAGAAAACAAGAUGAGAUUGGCAUGGCUUUAUUUGUU
UUUUUUGUUUUGUUUUGGUUUUUUUUUUUUUUUUGGCUUGACUCAGGAU
UUAAAAACUGGAACGGUGAAGGUGACAGCAGUCGGUUGGAGCGAGCAUCC
CCCAAAGUUCACAAUGUGGCCGAGGACUUUGAUUGCACAUUGUUGUUUUU
UUAAUAGUCAUUCCAAAUAUGAGAUGCGUUGUUACAGGAAGUCCCUUGCC
AUCCUAAAAGCCACCCCACUUCUCUCUAAGGAGAAUGGCCCAGUCCUCUCC
CAAGUCCACACAGGGGAGGUGAUAGCAUUGCUUUCGUGUAAAUUAUGUAA
UGCAAAAUUUUUUUAAUCUUCGCCUUAAUACUUUUUUAUUUGUUUUAUU
UUGAAUGAUGAGCCUUCGUGCCCCCCCUUCCCCCUUUUUUGUCCCCCAACU
UGAGAUGUAUGAAGGCUUUUGGUCUCCCUGGGAGUGGGUGGAGGCAGCCA
GGGCUUACCUGUACACUGACUUGAGACCAGUUGAAUAAAAGUGCACACCU
UAAAAAAAAAAAAAAAAAAAAAAAAAAAAAAAAAAAAAAAAAAAAAAAAA
AAUAACCCCUUGGGGC.

GAPDH mRNA, Human (produced by Nippon Gene Co., Ltd.)

GGGUUUAAAUUGAGCCCGCAGCCUCCCGCUUCGCUCUCUGCUCCUCCUGUU
CGACAGUCAGCCGCAUCUUCUUUUGCGUCGCCAGCCGAGCCACAUCGCUCA
GACACCAUGGGGAAGGUGAAGGUCGGAGUCAACGGAUUUGGUCGUAUUGG
GCGCCUGGUCACCAGGGCUGCUUUUAACUCUGGUAAAGUGGAUAUUGUUG
CCAUCAAUGACCCCUUCAUUGACCUCAACUACAUGGUUUACAUGUUCCAA

UAUGAUUCCACCCAUGGCAAAUUCCAUGGCACCGUCAAGGCUGAGAACGG
GAAGCUUGUCAUCAAUGGAAAUCCCAUCACCAUCUUCCAGGAGCGAGAUC
CCUCCAAAAUCAAGUGGGGCGAUGCUGGCGCUGAGUACGUCGUGGAGUCC
ACUGGCGUCUUCACCACCAUGGAGAAGGCUGGGGCUCAUUUGCAGGGGGG
AGCCAAAAGGGUCAUCAUCUCUGCCCCCUCUGCUGAUGCCCCCAUGUUCGU
CAUGGGUGUGAACCAUGAGAAGUAUGACAACAGCCUCAAGAUCAUCAGCA
AUGCCUCCUGCACCACCAACUGCUUAGCACCCCUGGCCAAGGUCAUCCAUG
ACAACUUUGGUAUCGUGGAAGGACUCAUGACCACAGUCCAUGCCAUCACU
GCCACCCAGAAGACUGUGGAUGGCCCCUCCGGGAAACUGUGGCGUGAUGG
CCGCGGGGCUCUCCAGAACAUCAUCCCUGCCUCUACUGGCGCUGCCAAGGC
UGUGGGCAAGGUCAUCCCUGAGCUGAACGGGAAGCUCACUGGCAUGGCCU
UCCGUGUCCCCACUGCCAACGUGUCAGUGGUGGACCUGACCUGCCGUCUA
GAAAAACCUGCCAAAUAUGAUGACAUCAAGAAGGUGGUGAAGCAGGCGUC
GGAGGGCCCCCUCAAGGGCAUCCUGGGCUACACUGAGCACCAGGUGGUCU
CCUCUGACUUCAACAGCGACACCCACUCCUCCACCUUUGACGCUGGGGCUG
GCAUUGCCCUCAACGACCACUUUGUCAAGCUCAUUUCCUGGUAUGACAAC
GAAUUUGGCUACAGCAACAGGGUGGUGGACCUCAUGGCCCACAUGGCCUC
CAAGGAGUAAGACCCCUGGACCACCAGCCCCAGCAAGAGCACAAGAGGAA
GAGAGAGACCCUCACUGCUGGGGAGUCCCUGCCACACUCAGUCCCCCACCA
CACUGAAUCUCCCCUCCUCACAGUUGCCAUGUAGACCCCUUGAAGAGGGG
AGGGGCCUAGGGAGCCGCACCUUGUCAUGUACCAUCAAUAAAGUACCCUG
UGCUCAACCAAAAAAAAAAAAAAAAAAAAAAAAAAAAAAAAAAAAAAAAA
AAAAAAAAAAUAACCCCUUGGGGC.

### Example 2: Difference in amplification effect due to difference in reaction temperature during the second strand synthesis

### 1. Acquisition of Ago2 binding RNA derived from HeLa cells

### (1) Preparation of an anti-human Ago2 antibody immobilized carrier

After PBS (pH 7.4) (1 mL) was added to 20 µL of Dynabeads ProteinG magnetic carrier (produced by Invitrogen Inc.) and mixed, the carrier was taken out using a magnetic stand. Thereafter, a mixed solution of 1 mg/mL anti-human Ago2 antibody (produced by Wako Pure Chemical Industries, Ltd.) (5 µL) and phosphate buffer saline (PBS, pH 7.4) (95 µL) was added to the carrier, and blended by tumble mixing at room temperature for 1 hour. Subsequently, the carrier was taken out using a magnetic stand, washed with PBS (pH 7.4) (1 mL) 3 times, and finally suspended with PBS (pH 7.4) (1 mL). Resulting solution was used as an anti-human Ago2 antibody immobilized carrier solution.

### (2) Preparation of HeLa cells extraction liquid

A cell liquid (0.05w/v% NP-40 containing 20 mM Tris-HCl, 200 mM sodium chloride and 2.5 mM magnesium chloride (produced by Wako Pure Chemical Industries, Ltd.) (1 mL) was added to HeLa cells (1×10⁷ cells), and cells were suspended by pipetting. The suspension was allowed to stand on ice for 10 minutes. After centrifugation (20000 × g, at 4°C for 20 minutes), the supernatant was fractionated, and the resulting liquid was used as HeLa cells extraction liquid.

### (3) Purification of RNA derived from HeLa cells

After the carrier was taken out from the anti-human Ago2 antibody immobilized carrier solution using a magnetic stand, HeLa cells extraction liquid (1 mL) was added, and blended by tumble mixing under refrigeration for 3 hours. After the carrier was taken out again using a magnetic stand, and washed with cell lysate (1 mL) 3 times. Thereafter, 0.5w/v% dodecyl sodium sulfate (SDS) solution (50 µL) was added to the carrier, protein bound to the carrier was eluted. Sterilized water (350 µL) and phenol: chloroform : isoamyl alcohol(25 : 24 : 1) (400 µL) were added to the resulting eluate, and mixed with a vortex mixer, and then centrifugation (20000 × g, for 10 minutes) was carried out. Subsequently, the upper layer was fractionated, chloroform (400 µL) was added thereto, mixed with a vortex mixer, and centrifugation (20000 × g, for 10 minutes) was carried out. Further, the upper layer was fractionated, and Ethachinmate (produced by Nippon Gene Co., Ltd.) (3 µL), 3 M sodium acetate (40 µL), and ethanol (1 mL) were added thereto, and suspended with a vortex mixer, and then centrifugation (20000 × g, for 15 minutes) was carried out. The resulting precipitate was washed with 70v/v% ethanol (1 mL), air-dried at room temperature for 20 minutes, and dissolved in sterilized water (11 µL) to obtain a purified RNA solution.

### 2. Reverse transcription reaction

The RNA solution (11 µL) obtained by the above-described anti-human Ago2 antibody immobilized carrier solution and 20 µM oligo(dT)primer solution (5'-GACCATATGACGAGATCCGAGCTTCTTTTTTTTTTTTTTTTTTTT-3') (1 µL) were added and mixed. The mixed solution was incubated at 70°C for 3 minutes, and then cooled down on ice. Subsequently, 2.5 mmol/L dNTPs mixed solution (produced by Nippon Gene Co., Ltd.) (4 µL), 20 U/µL RNase Inhibitor Super (produced by Wako Pure Chemical Industries, Ltd.) (1 µL), 10 × reaction buffer solution for ReverScript IV (produced by Wako Pure Chemical Industries, Ltd.) (2 µL), and 200 U/µL ReverScript IV (reverse transcriptase, produced by Wako Pure Chemical Industries, Ltd) (1 µL) were added and mixed thereto, and the reaction liquid (20 µL in total volume) was reacted at 42°C for 10 minutes. Thereafter, the reaction was terminated by adding 0.5 mol/L EDTA (2 µL) and mixing sufficiently.

### 3. Alkali treatment

After termination of the reaction, 0.2 mol/L sodium hydroxide (8 µL) was added thereto and mixed, heated at 65°C for 30 minutes, and then allowed to stand on ice for 2 minutes. Further, after 1 mol/L Tris-HCl (pH 7.5) (20 µL) was added thereto and mixed, unreacted reverse transcription reaction primer was removed using Invisorb Spin PCRapid Kit (produced by Invitek Inc.). Subsequently, Ethachinmate (produced by Nippon Gene Co., Ltd.) (1 µL), 10 mol/L ammonium acetate (12 µL), and ethanol (125 µL) were added, respectively, and mixed. Further, the mixture was centrifuged (18,800 × g) for 10 minutes, respectively, washed with 75% ethanol twice, precipitated and dried, and then dissolved in sterilized water (35.5 µL).

### 4. Second strand synthesis reaction

To the aqueous solution containing the reverse transcription reaction product (35.5 µL) obtained in the above-described item 3, 10 × Reaction Buffer for HotGoldstar DNA polymerase (produced by EUROGENTEC Inc.) (5 µL), each of 2.5 mmol/L dNTP Mixture (produced by Nippon Gene Co., Ltd.) (4 µL), 20 µmol/L random primer solution for second strand synthesis (5'-CATGGTATCGACGAGACTGAGGCTGNNNNNNNNNB-3', B represents C, G or T, and N represents A, C, G or T) (1 µL), 25 mmol/L MgCl₂ (4 µL), and HotGoldstar DNA polymerase (5U/µL, produced by EUROGENTEC Inc.) (0.5 µL) were added, respectively, mixed gently on ice, and second strand synthesis reaction was carried out under the following conditions:
95°C for 10 minutes → 30°C for 1 minute →0°C for 30 seconds → 0°C for 30 seconds → 60°C for 30 seconds → 72°C for 2 minutes → 95°C for 1 minute → on the ice for 2 minutes.

In addition, the second strand synthesis reaction was carried out in the same way as in the above-described reaction, except that the aqueous solution containing the reverse transcription reaction products (35.5 µL) obtained by repeating the same reaction as in the above-described items 1 to 3 was used and the second strand synthesis reaction was carried out under the following conditions:
95°C for 10 minutes → 30°C for 1 minute → 72°C for 2 minutes → 95°C for 1 minute → on the ice for 2 minutes.

Subsequently, unreacted reverse transcription reaction primer was removed from each of the resulting solutions by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125 µL) were added, respectively, and mixed. Further, each mixture was centrifuged (18,800 × g), washed with 75% ethanol twice, and each precipitate was dried and dissolved in sterilized water (34.5µL).

### 5.PCR

To each of 2 kinds of aqueous solution obtained in the above item 4 (34.5 µL each), 10 × Reaction Buffer for HotGoldstar DNA polymerase (EUROGENTEC Inc.) (5 µL), 2.5 mmol/L dNTP Mixture (produced by Nippon Gene Co., Ltd.) (4 µL), PCR primer solution (20 µmol/L, 5'-CATGGTATCGACGAGACTGAG-3' and 5'-GACCATATGACGAGATCCGAG-3') (1µL each), 25 mmol/L MgCl₂ (4 µL), and HotGoldstar DNA polymerase (5U/µL, produced by EUROGENTEC Inc.) (0.5 µL) were added, respectively, mixed gently on ice, and PCR reaction was carried out under the following conditions:
95°C for 10 minutes → 95°C for 20 seconds · 60°C for 10 seconds · 72°C for 30 seconds (24 cycles) → 72°C for 2 minutes.

Subsequently, unreacted PCR primer was removed from each resulting solution by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125 µL) were added, respectively, and mixed. Further, each mixture was centrifuged for 10 minutes (18,800 × g), washed with 75% ethanol twice, and precipitate was dried, and then dissolved in sterilized water (5 µL).

As for 1 µL each of the resulting solutions, chain length analysis was carried out by using Agilent 2100 Bioanalyzer. Results are shown in the following Table 2.

**[Table 2]**

| | Annealing temperature (30°C →72°C) | Annealing temperature (30°C→ 40°C → 50°C →60°C→72°C) |
|---|---|---|
| Chain length [bp] | RNA synthesis amount (ng/µl) | RNA synthesis amount (ng/µl) |
| 203 | 6.91 | 9.12 |
| 224 | 5.83 | 14.8 |

An amount of the intended RNA synthesized was 12.74 ng (6.91 ng + 5.83 ng) for the case of annealing temperature (30°C → 72°C), 23.92 ng (9.12 ng + 14.8 ng) for the case of annealing temperature (30°C → 40°C → 50°C → 60°C → 72°C), and it was found that the amount of DNA to be synthesized was increased when temperature was increased in a stepwise manner, like 30°C → 40°C → 50°C → 60°C → 72°C. However, it was also found that even when temperature was increased in 2 steps like 30°C → 72°C, DNA was synthesized and amplified in such amount that DNA analysis could be carried out sufficiently.

### Example 3: Synthesis of DNA corresponding to RNA not having Cap structure (Alu RNA derived from HeLa cell)

### 1. Transformation

To the PCR reaction solution (2 µL) obtained in the above item 5 in Example 2, 20 ng/µL pGEM Teasy Vector (produced by Promega Inc.) (1 µL) and DNA Ligation Kit Mighty Mix solution (produced by TAKARA Biotech Co., Ltd.) (3 µL) were added, and the mixture was reacted at 16°C for 30 minutes, to insert the cDNA for cloning into the vector. Thereafter, the total volume was transformed into competent cells using ECOS™ Competent E. coli DH5α (produced by Nippon Gene Co., Ltd.) by the heat shock method at 42°C for 45 seconds. Subsequently, the competent cells were incubated in the Luria-Bertani's broth (LB) agar media containing 100 µg/mL of ampicillin sodium at 37°C for 16 hours.

### 2. Colony PCR

To a part of each single colony on the media, the reaction solution [total volume (10 µL): 10 × Universal Buffer (produced by Nippon Gene Co., Ltd.) (1 µL), 2.5 mM dNTPs (a mixed solution of 2.5 mmol/L each of dATP, dGTP, dCTP, dTTP, produced by Nippon Gene Co., Ltd.) (1 µL), 5 µmol/L of 2 kinds of primers (5'-GTATCGACGAGACTGAGGCTG-3',
5'-GAAGCACAGAGAAAAGAGGCAAAATG-3') (1 µL each), sterilized water (5.9 µL), Gene Taq NT (5 units/µL, produced by Nippon Gene Co., Ltd.) (0.1 µL)] was added, and the mixture was mixed gently on ice, and PCR reaction was carried out under the following conditions.
95°C for 2 minutes → 95°C for 20 seconds · 58°C for 10 seconds · 72°C for 20 seconds (30 cycles) → 72°C for 1 minute.

After that, 6 × Loading Buffer Triple Dye (produced by Nippon Gene Co. Ltd.) (2 µL each) were added to each of the resulting PCR reaction solutions and mixed. Positive clones were selected by electrophoresis, and plasmid extraction was carried out.

### 3. Nucleotide sequence analysis

Nucleotide sequence analysis for the above-described plasmid was carried out by BaseStaion (produced by Bio-Rad Laboratories, Inc.), and was compared with the database registered in NCBI.

As a result, it was found that there were 4 kinds of cDNAs sharing 80% or more of homology with Alu RNA. Alu RNA has been considered to function as retrotransposon, and assumed to bind to Ago2. In addition, Alu RNA is RNA not having Cap structure. That is to say, it was proved from this experiment that there was Alu RNA in Ago2, and it was found that according to the method of the present invention, DNA corresponding to the Alu RNA not having Cap structure could be also obtained (amplified). The analysis results of the resulting 4 kinds of Alu RNAs are shown below.

free Alu RNA 1 (Homology with Human primary Alu transcript (GeneBank accession No. U67811) : 86%)

TTCAAGGGGTTTGGCTTTGTTCGTTTTACGGAATATGAAACACAAGTGAAAGT
AATGTCACAGCGACATATGATAGATGGACGATGGTGTGACTGCAAACTTCCTA
ATTCTAAGCAAAGCCAAGATAAGCCTTTGAGAAGCAGAAAAGTGTTTGTGGG
GCGCTGTACAGAGGACATGACTGAGGATGAGCTGCGGGAGTTCTTCTCTCAGT
ACGGGGATGTGATGGATGTCTTCATCCCCAAGCCATTCAGGGCCTTTGCCTTT
GTTACATTTGCAGATGATCAGGTATTTTTCTCCTTAACGATATGTCCCGGCCGG
GCGTGGTGGCTCATGCTTACAATCCCAGCACTTTGGGAGGCCGAGGCGGGTG
GATCACGAGGTCAGGAGATCGGGACCATCCTGGCCAGCATGGTGAAACCCCG
TCTCTATT.

free Alu RNA 2 (Homology with Human primary Alu transcript (GeneBank accession No. U67812) : 83%)

GAAAATGAGGTCAGGAGTTTGGGACCAGCCTGGCCAACATGGTGAACCTCTG
TCTCTACTAAAAATACAAAAATTAGCCAAGTGTGGTGGTGCGTGCCTGTAATC
CCAGCCTCTCGGGAGACTGAGGCAGGAGAATTGCTTGAACCCAGGAGGCGG
AGGTTGCAGTGAGCTGAGGTCGTGCCACTGCACTCCAGCCTGGGCAATAGGG
CAAGACTCTGTCTC.

free Alu RNA 3 (Human primary Alu transcript (Homology with GeneBank accession No. U67813) : 84%)

GGGTAGTGCTTGAGCCTAGAAGTTGCAGTGAGCTATGATCATGCCACACTGTA
CTCCAGCCTGGATGACAGAGTGAAACCCTGCCTCTAAATAAAAGAAAATAGA

GGCAGACTGTGGTGGCTCACGCCTGTAATCCCAGCACTTTGGTAGGCCAAGG
CGGGTGGATCACCTGCAGTCAGGAGTTCACAACCAGCCTGACCAACATGGTG
AAACCCTGTCTATACTAAAAATACAAAACATTAGCCACGTGTGGTGGTACACG
CCTGTAATCCCAGCTACTCGGGAGGCTGAGTCAGGAGAATCACTTGAACCTG
GGAGGCGGAGGTTGCAGTGAGCTGAGATTGTACCACTGCACTCCAGCCTGGG
TGACAGAGCAAAACCCTATCTC.

free Alu RNA 4 (Human primary Alu transcript (Homology with GeneBank accession No. U67812) : 86%)

AACATCGCTTGAACCTGGGAGGTGGAGGTTGCAGTGAGCAGAGATCGCGCCA
TTGCACTCCAGCCTGGGCAATAGAGTGAGACCCTGTCTCAAAAAAAAGAAAA
AGAAAAAGAAAAGAAAGGGACTGCAAACTCTAACAACCTTCAATATGTCCTT
GCTCAGATCCATTTTAAAAATTATATTCATGTGTTTTGTAAACATATCATTTCTG
GG.

### Example 4: Synthesis of cDNA of PIWI binding mRNA

### 1. Acquisition of PIWI binding RNA derived from mouse testis

### (1) Preparation of anti-mouse PIWII,1 antibody and mouse IgG immobilized carrier

To a magnetic carrier Dynabeads Protein G (produced by Invitrogen Inc.) (20 µL), PBS (pH 7.4) (1 mL) was added and mixed, and the carrier was taken out using a magnetic stand. Thereafter, a mixed solution of 0.5 mg/mL anti-mouse PIWIL1 antibody (produced by Wako Pure Chemical Industries, Ltd.) (10 µL) and PBS (pH 7.4) (90 µL) was added to the carrier, and the mixture was blended by tumble mixing at room temperature for 1 hour. Subsequently, the carrier was taken out using a magnetic stand, washed 3 times with PBS (pH 7.4) (1 mL), to obtain an anti-mouse PIWIL1 antibody immobilized carrier.

In addition, an anti-mouse IgG antibody immobilized carrier was obtained in the same manner as above, except that a mixed solution of 1 mg/mL mouse IgG antibody (produced by Wako Pure Chemical Industries, Ltd.) (5 µL) and PBS (pH 7.4) (95 µL) was employed instead of instead of the mixed solution of 0.5 mg/mL anti-mouse PIWIL1 antibody (Wako Pure Chemical Industries, Ltd.) (10 µL) and PBS (pH 7.4) (90 µL).

### (2) Preparation of mouse testis extract

To the testis (25 mg) isolated from Balb/c mouse (adult, male), cell lysate (0.05w/v% NP-40 containing 20 mM Tris-HCl, 200 mM sodium chloride, and 2.5 mM magnesium chloride (produced by Wako Pure Chemical Industries, Ltd.)) (1 mL) was added, and cells were suspended using a Teflon (registered trademark) homogenizer. The suspension was allowed to stand on ice for 10 minutes, centrifuged (20000 × g, at 4°C for 20 minutes), and then the supernatant was fractionated, and filtered with a 0.45 µm filter. The filtrate was used as a mouse testis extract.

### (3) Purification of PIWI binding RNA derived from mouse testis by immunoprecipitation method

Mouse testis extract (1 mL) was added to anti-mouse PIWIL1 antibody, and was blended by tumble mixing under refrigeration for 3 hours. The carrier was taken out using a magnetic stand, and then washed with cell lysate (1 mL) 3 times. Thereafter, 0.5w/v% SDS solution (50 µL) was added to the carrier, and a protein bound to the carrier was eluted. Sterilized water (350 µL), and a mixed solvents of phenol : chloroform : isoamyl alcohol (25:24:1) (400 µL) were added to the resulting eluate, and the mixture was mixed with a vortex mixer, and then centrifuged (20000 × g, for 10 minutes). Subsequently, the upper layer was fractionated, chloroform (400 µL) was added thereto, and the mixture was mixed with a vortex mixer, and then centrifuged (20000 × g, for 10 minutes). Further, the upper layer was fractionated, and Ethachinmate (produced by Nippon Gene Co., Ltd.) (3 µL), 3 M sodium acetate (40 µL), and ethanol (1 mL) were added thereto, and the mixture was suspended with a vortex mixer, and then centrifuged (20000 × g, for 15 minutes). The resulting precipitate was washed with 70v/v% ethanol (1 mL), air dried at room temperature for 20 minutes, and then dissolved in sterilized water (11 µL), to obtain a purified RNA solution.

In addition, a purified RNA solution as a blank was obtained in the same way as in the case where anti-mouse PIWIL1 antibody was used, using the mouse IgG immobilized carrier, adding and blending mouse testis extract, eluting a protein from the carrier with SDS solution, and purifying the eluate using a mixed solvent of phenol : chloroform : isoamyl alcohol (25 : 24 : 1) and Ethachinmate.

### 2. Reverse transcription reaction

To the purified RNA solution (11 µL) obtained from anti-mouse PIWIL1 antibody and the purified RNA solution (11 µL) obtained from the mouse IgG immobilized carrier, a 20 µM oligo(dT)primer solution (5'-GACCATATGACGAGATCCGAGCTTCTTTTTTTTTTTTTTTTTTTT-3') (1 µL) was added and mixed, respectively. Each mixed solution was incubated at 70°C for 3 minutes, and then cooled down on ice. Subsequently, 2.5 mmol/L dNTPs mixed solution (produced by Nippon Gene Co., Ltd.) (4 µL), 20 U/µLRNase Inhibitor Super (produced by Wako Pure Chemical Industries, Ltd.) (1 µL), 10 × reaction buffer solution for ReverScript IV (produced by Wako Pure Chemical Industries, Ltd.) (2 µL), and 200 U/µL ReverScript IV (reverse transcriptase, produced by Wako Pure Chemical Industries, Ltd) (1 µL) were added and mixed thereto, respectively, and 2 kinds of the reaction liquids (20 µL in total volume) was reacted at 42°C for 10 minutes. Thereafter, the reaction was terminated by adding 0.5 mol/L EDTA (2 µL) and mixing sufficiently.

### 3. Alkaline treatment

After termination of the reaction, 0.2 mol/L sodium hydroxide (8µL) was added and mixed, respectively, heated at 65°C for 30 minutes, and then allowed to stand on ice for 2 minutes. Further, after 1 mol/L Tris-HCl (pH 7.5) (20 µL) was added and mixed, respectively, unreacted reverse transcription reaction primer was removed using Invisorb Spin PCRapid Kit (produced by Invitek Inc.). Subsequently, Ethachinmate (produced by Nippon Gene Co., Ltd.) (1 µL), 10 mol/L ammonium acetate (12 µL), and ethanol (125 µL) were added, respectively, and mixed. Further, each mixture was centrifuged (18,800 × g) for 10 minutes, washed with 75% ethanol twice, precipitated and dried, and then dissolved in sterilized water (35.5 µL).

### 4. Second strand synthesis reaction

To the 2 kinds of aqueous solution containing the reverse transcription reaction product (35.5 µL), 10 × Reaction Buffer for HotGoldstar DNA polymerase (produced by EUROGENTEC Inc.) (5 µL), each of 2.5 mmol/L dNTP Mixture (produced by Nippon Gene Co., Ltd.) (4 µL), 20 µmol/L random primer solution for second strand synthesis (5'-CATGGTATCGACGAGACTGAGGCTGNNNNNNNNNB-3', B represents C, G or T, and N represents A, C, G or T) (1 µL), 25 mmol/L MgCl₂ (4 µL), and HotGoldstar DNA polymerase (5U/µL, produced by EUROGENTEC Inc.) (0.5µL) were added, respectively, mixed gently on ice, and second strand synthesis reaction was carried out under the following conditions.
95°C for 10 minutes → 30°C for 1 minute → 40°C for 30 seconds → 50°C for 30 seconds → 60°C for 30 seconds → 72°C for 2 minutes → 95°C for 1 minute → on the ice for 2 minutes.

Subsequently, unreacted reverse transcription reaction primer was removed from each of the resulting solutions by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125 µL) were added and mixed, respectively. Further, each mixture was centrifuged (18,800 × g), washed with 75% ethanol twice, and each precipitate was dried and dissolved in sterilized water (34.5 µL).

5.PCR

To each of aqueous solution obtained in the above item 4 (34.5µL each), 10 × Reaction Buffer for HotGoldstar DNA polymerase (EUROGENTEC Inc.) (5 µL), each of 2.5 mmol/L dNTP Mixture (produced by Nippon Gene Co., Ltd.) (4µL), PCR primer solution (20 µmol/L, 5'-CATGGTATCGACGAGACTGAG-3' and 5'-GACCATATGACGAGATCCGAG-3') (1 µL each), 25 mmol/L MgCl₂ (4 µL), and HotGoldstar DNA polymerase (5U/µL, produced by EUROGENTEC Inc.) (0.5µL) were added, respectivley, mixed gently on ice, and PCR reaction was carried out under the following conditions.
95°C for 10 minutes → 95°C for 20 seconds · 60°C for 10 seconds · 72°C for 30 seconds (24 cycles) → 72°C for 2 minutes.

Subsequently, unreacted PCR primer was removed from each resulting solution by using Invisorb Spin PCRapid Kit (produced by Invitek Inc.). Thereafter, Ethachinmate (produced by Nippon Gene Co., Ltd.) (1 µL), 10 mol/L ammonium acetate (12 µL), and ethanol (125 µL) were added and mixed, respectively. Further, each mixture was centrifuged for 10 minutes (18,800 × g), washed with 75% ethanol twice, and precipitate was dried, and then dissolved in sterilized water (5 µL).

For the solution ( 1 µL) obtained by employing the anti-PIWIL1 antibody-immobilized carrier, capillary electrophoresis was carried out using Agilent 2100 Bioanalyzer. Results thereof are shown in Fig. 3. In addition, results of capillary electrophoresis by Agilent 2100 Bioanalyzer for the solution ( 1µL) obtained by using anti-IgG antibody-immobilized carrier as a blank are shown in Fig. 4.

From the results in Fig. 3 and Fig. 4, it can be understood that cDNA within a range of 100 to 1500 bp has been amplified by using anti-PIWIL1 antibody-immobilized carrier. That is, according to the present invention, it was shown that cDNA derived from traces of mRNAbound to PIWTL1 could be amplified.

### Example 5

Additional experiments were carried out in the same manner as in Example 1, and it was confirmed that the following mRNAs could be also identified according to the method of the present invention.
mRNA (GeneBank accession No.) : ALPI (J03930), ANKHD1 (BC009909), ATAD3A (BC063607), BASP1 (BC000518), BHLHB2 (EF015895), BLVRB (BC109371), C12orf10 (BC051871), COP1 (AY885669), CPNE1 (BC001142), CTNNBIP1 (BC014300), DC36 (AF265442), DKFZp451P176 (AL832365), DKFZp667O181 (AL832271), DKFZp686E13246 (CR627112), DKFZp686H2396 (BX648036), DKFZp686M18208 (BX648809), DNAJC11 (BC008772), FLJ12498 (AK022560), FLJ26394 (AK129904), FLJ30115 (AK054677), FLJ33729 (AK091048), FLJ33826 (AK091145), FLJ34030 (AK091349), FLJ36398 (AK093717), FLJ37703 (AK095022), FLJ41250 (AK123244), FTH1 (M11146), H19 (BC110657), HET (U72355), HMGN2 (BC081567), HSBP1 (AF068754), hypothetical protein (AJ011916), IMAGE:40148924 (BC142726), JDP2 (BC051303), KIAA0184 (D80006), LOC128977 (BC030758), MRPS24 (BC012167), NACAP1 (BC069411), NAT11 (BC052298), NDUFB2 (BC063026), NOLA2 (BC000009), PFN1 (BC057828), PNKD (B021118), PNPLA7 (BC025663), POLD3 (BC020587), PRDX6 (BC053550), PTPMT1 (BC014048), RNASEH2A (BC011748), RPL8 (BC093064), RPL22 (BC058887), RPL30, BC095426), RPLP1 (BC007590), RPS10 (BC073799), RPS20 (BC087850), RPS21 (L04483), RPS28 (BC070218), RPS29 (BC035313), S100A6 (BC009017), S100A11 (B014354), SFRS7 (BC017369), SOCS3 (BC060858), TMSB10 (BC016731), TUBG2 (BC108739), ZNF205 (B002810).

## Claims

1. A method for synthesis of a double-stranded DNA having a nucleotide sequence corresponding to template RNA having polyA, comprising:
1) step 1 in which reverse transcription reaction of template RNA is carried out employing oligo(dT)primer to which DNA fragment having a known sequence has been added at the 5'-terminal, to obtain a single-stranded DNA; and
2) step 2 in which double strand formation reaction of the single-stranded DNA obtained in step 1 is carried out employing random primer to which DNA fragment having a known sequence has been added at the 5'-terminal, in the presence of polymerase which does not have 3'→5' exonuclease activity nor strand displacement activity, to obtain a double-stranded DNA.

2. The synthesis method according to claim 1, further comprising a step in which the obtained single-stranded DNA is purified after step 1.

3. The synthesis method according to claim 1, wherein chain length of the RNA having polyA is 30 to 1500 bases.

4. The synthesis method according to claim 1, wherein chain length of the random primer is 5 to15mer.

5. The synthesis method according to claim 1, wherein the double strand formation reaction in step 2 is carried out by reacting at 90 to 98°C for 1 to 15 minutes, at 20 to 40°C for 10 seconds to 5 minutes, and at 65 to 75°C for 10 seconds to 10 minutes, in this order.

6. A method for amplification of a double-stranded DNA having a nucleotide sequence corresponding to template RNA having polyA, comprising:
1) step 1 in which reverse transcription reaction of template RNA having polyA is carried out employing an oligo(dT)primer to which DNA fragment having a known sequence (adapter 1) has been added at the 5'-terminal, to obtain a single-stranded DNA;
2) step 2 in which double strand formation reaction of the single-stranded DNA obtained in step 1 is carried out employing a random primer to which DNA fragment having a known sequence (adapter 2) has been added at the 5'-terminal, in the presence of polymerase which does not have 3'→5' exonuclease activity nor strand displacement activity, to obtain a double-stranded DNA; and
3) step 3 in which PCR reaction is carried out by using the double-stranded DNA obtained in step 2 as a template, employing primer 1 having the nucleotide sequence of adapter 1 at the 3'-terminal and primer 2 having the nucleotide sequence of adapter 2 at the 3'-terminal.

7. The amplification method according to claim 6, further comprising a step in which the obtained single-stranded DNA is purified after step 1.

8. The amplification method according to claim 6, wherein chain length of the RNA having polyA is 30 to 1500 bases.

9. The amplification method according to claim 6, wherein chain length of the random primer is 5 to15mer.

10. The amplification method according to claim 6, wherein the double strand formation reaction in step 2 is carried out by reacting at 90 to 98°C for 1 to 15 minutes, at 20 to 40°C for 10 seconds to 5 minutes, and at 65 to 75°C for 10 seconds to 10 minutes, in this order.
